# EUROPÄISCHE PATENTSCHRIFT

(11) **EP 0 795 536 B1**
(45) Veröffentlichungstag und Bekanntmachung des Hinweises auf die Patenterteilung: **25.07.2001**
(21) Anmeldenummer: 97101739.7
(22) Anmeldetag: 04.02.1997
(51) Int. Cl.: C07C 67/08, C07C 67/54, C07C 69/54

(54) **Verfahren zur kontinuierlichen Herstellung von Alkylestern der (Meth)acrylsäure**
Process for the continuous preparation of alkyl esters of (meth)acrylic acid
Procédé de fabrication en continue d'esters alkyliques d'acide (méth)acrylique

(30) Priorität: 06.02.1996 DE 19604253
(43) Veröffentlichungstag der Anmeldung: 17.09.1997
(73) Patentinhaber: BASF AKTIENGESELLSCHAFT, 67056 Ludwigshafen (DE)
(72) Erfinder: Schmidt, Willi, 67069 Ludwigshafen (DE); Geisendörfer, Matthias, Dr., 67435 Neustadt (DE); Dockner, Toni, Dr., 67149 Meckenheim (DE); Herbst, Holger, Dr., 67227 Frankenthal (DE); Nestler, Gerhard, Dr., 67061 Ludwigshafen (DE); Weck, Alexander, 67251 Freinsheim (DE)
(74) Vertreter: Isenbruck, Günter, Dr.

(56) Entgegenhaltungen:
- EP-A- 0 694 524
- DE-A- 2 552 987

## Beschreibung

Die Erfindung betrifft ein Verfahren zur kontinuierlichen Herstellung von Alkylestern der (Meth)acrylsäure durch Umsetzung von (Meth)acrylsäure mit 4 bis 8 C-Atome aufweisenden Alkanolen in homogener, flüssiger, an Lösungsmittel freier Phase bei erhöhter Temperatur und in Gegenwart eines sauren Veresterungskatalysators.

Bei Veresterungen von Alkanol mit organischer Säure laufen generell typische Gleichgewichtsreaktionen ab, die durch starke Säuren katalysiert werden und die als typische Kondensationsreaktionen zur Abspaltung von Wasser führen. Üblicherweise wird durch Entfernung des Wassers aus dem Reaktionsgemisch das Veresterungsgleichgewicht in die gewünschte Richtung verschoben. Die Abtrennung des Wassers kann destillativ als Bestandteil eines Azeotrops erfolgen, das auch den Zielester umfaßt. Mit der kontinuierlichen Abtrennung des Reaktionswassers aus dem Reaktionsgemisch geht dann gleichzeitig die Abtrennung des Zielesters vom Reaktionsgemisch einher. In der Regel erfolgt die Veresterungsreaktion aber so, daß zwar das Wasser kontinuierlich aus dem Reaktionsgemisch entfernt wird, die Hauptmenge des gebildeten Zielesters aber im Reaktionsgemisch verbleibt.

Beispiele für Veresterungen dieser Art bilden diejenigen, bei denen das Reaktionswasser durch Zusatz eines organischen Lösungsmittels als azeotropem Schlepper destillativ abgetrennt wird. Als ein solches azeotropes Schleppmittel kann aber auch im Überschuß eingesetztes (Ausgangs)alkanol dienen. Eine andere Variante besteht darin, daß das Wasser als Bestandteil eines Heteroazeotrops aus Zielester/Alkanol/Wasser destillativ abgetrennt wird, wobei die organische Phase im wesentlichen vollständig in die Veresterung zurückgeführt wird.

Die bei solchen Veresterungen entstehenden Produktgemische enthalten im wesentlichen den gebildeten Alkylester der (Meth)acrylsäure, den sauren Veresterungskatalysator und im Verlauf der Veresterung gebildete höher als der Alkylester der (Meth)acrylsäure siedende Nebenprodukte. Ferner enthalten die Produktgemische in der Regel Polymerisationsinhibitoren sowie gegebenenfalls Bestandteile aus der Gruppe umfassend gegebenenfalls überschüssiges Alkanol, überschüssige (Meth)acrylsäure, azeotrope Schleppmittel, organische Lösungsmittel sowie Restmengen an Wasser. Aus diesen Produktgemischen muß dann der Zielester abgetrennt werden. Gemäß Ullmann's Encyclopedia of Industrial Chemistry, 5th edition, Vol. A1, VCH Weinheim, Seite 168/169 erfolgt diese Abtrennung in der Regel so, daß das Produktgemisch zunächst mit Wasser gewaschen wird. Der saure Veresterungskatalysator und die überschüssige Ausgangssäure gehen aus der organischen Produktgemischphase in die Wasserphase und werden so aus dem Produktgemisch abgetrennt. Durch Nachwaschen mit wäßriger Alkalilauge wird diese Abtrennung normalerweise vervollständigt.

Aus der verbleibenden organischen Phase werden anschließend in einer ersten Rektifikationskolonne regelmäßig zunächst das verbliebene Alkanol und danach in einer weiteren Rektifikationskolonne der Zielester jeweils über Kopf abgetrennt.

Die Nachteile einer solchen Aufarbeitungsweise liegen insbesondere im Anfall von großen Mengen stark belasteten Abwassers. Außerdem sind die in der wäßrigen Alkalilauge gelöste Ausgangssäure und das darin gelöste Alkanol in der Regel nicht direkt und technisch aufwendig in die Veresterung zurückführbar, was Eduktverluste bedingt.

Ein wasserfreies Aufarbeitungsverfahren ist beispielsweise aus der DE-C 25 48 561 für die Herstellung von 2-Ethylhexylacrylat bekannt. Bei der dort beschriebenen Aufarbeitungsweise werden überschüssiges Alkanol und überschüssige Ausgangssäure über Kopf vom Produktgemisch destillativ abgetrennt. In einer nachfolgenden Destillationskolonne wird dann aus dem Sumpfprodukt der vorhergehenden Destillationskolonne der Zielester destillativ abgetrennt. Das Sumpfprodukt, aus dem die destillative Abtrennung des Zielesters erfolgt, enthält dabei noch den sauren Katalysator der eigentlichen Veresterungsreaktion. Außerdem erfordert die destillative Abtrennung des Zielesters erhöhte Temperaturen auch bei reduziertem Druck. Dies führt gemäß eigenen Untersuchungen dazu, daß eine Spaltung der im Rahmen der eigentlichen Veresterung als Nebenprodukte gebildeten höher als der Alkylester der (Meth)acrylsäure siedenden Verbindungen in leichtersiedende Bestandteile auftritt, so daß die Reinheit eines so gewonnenen Zielesters nicht befriedigt.

Der Erfindung liegt die Aufgabe zugrunde, ein Verfahren zur kontinuierlichen Herstellung von Alkylestern der (Meth)acrylsäure zu schaffen, das neben einer optimierten Ausbeute mildere Reaktionsbedingungen und damit neben stark verminderter Etherbildung weniger Hochsiederbildung, eine hohe Raum-Zeit-Ausbeute, eine erhöhte Flexibilität des Betriebs der Anlage sowie niedrige Investitionskosten aufgrund einer minimierten Apparatezahl ermöglicht.

Die Lösung geht aus von dem bekannten Verfahren zur kontinuierlichen Herstellung von Alkylestern der (Meth)acrylsäure durch Umsetzung von (Meth)acrylsäure mit 4 bis 8 C-Atome aufweisenden Alkanolen in homogener, flüssiger, an Lösungsmittel freier Phase bei erhöhter Temperatur und in Gegenwart eines sauren Veresterungskatalysators, bei dem man die (Meth)acrylsäure, das Alkanol und den Katalysator einer Reaktionszone zuführt, während einer Verweilzeit das gebildete Wasser als Bestandteil eines Alkanol umfassenden Gemisches in einer der Reaktionszone aufgesetzten Rektifikationseinheit rektifikativ abtrennt, das dabei anfallende Destillat in eine Alkanol enthaltende organische und in eine Wasser enthaltende wäßrige Phase auftrennt, die organische Phase mit Ausnahme eines Teilstroms, der ausgeschleust wird in die Rektifikationseinheit zurückführt, das Reaktionsgemisch aus der Reaktionszone austrägt und in eine weitere Rektifikationseinheiten umfassende destillative Trennzone leitet und in dieser den gebildeten Alkylester der (Meth)acrylsäure abtrennt.

Das erfindungsgemäße Verfahren ist dann dadurch gekennzeichnet, daß
a) (Meth)acrylsäure und Alkanol im Molverhältnis von 1:0,75 bis 1:2 umgesetzt werden,
b) von der am Kopf der Rektifikationseinheit III anfallenden wäßrigen Phase ein Teil in die Rektifikationseinheit zurückgeführt wird,
c) das aus der Reaktionszone ausgetragene Reaktionsgemisch einer weiteren Rektifikationseinheit I zugeführt und in dieser das Reaktionsgemisch in ein den Katalysator enthaltendes Produkt II und in ein den Alkylester der (Meth)acrylsäure, verbliebenen Alkanol und verbliebene (Meth)acrylsäure enthaltendes Produkt I aufgetrennt wird, und
d) das Produkt I einer weiteren Rektifikationseinheit II zugeführt und in dieser der Alkylester der (Meth)acrylsäure vom verbliebenen Alkanol und von der verbliebenen (Meth)acrylsäure abgetrennt und das verbliebene Alkanol und die verbliebene (Meth)acrylsäure in die Reaktionszone zurückgeführt werden.

Der Begriff Rektifikationseinheit ist hier wie auch im folgenden als allgemeine Bezeichnung für Apparate zu verstehen, in denen durch Wärmezufuhr Dämpfe erzeugt werden, die aufsteigen und in Kontakt mit abströmender flüssiger Phase stehen. In der Regel handelt es sich hierbei um Rektifikationskolonnen, in denen Einbauten für den intensiven Kontakt zwischen Flüssigkeit und Dampf enthalten sind. Derartige Einbauten sind Böden, wie Glockenböden, Lochböden, insbesondere Dual-Flow-Böden, Schüttungen, Packungen oder dergleichen.

Zur Vereinfachung des Verständnisses der Zusammenhänge sind die verschiedenen Rektifikationseinheiten mit römischen Ziffern bezeichnet. Auch die verschiedenen, im einzelnen beschriebenen Produkte tragen eine derartige Bezeichnung.

Die Reaktionszone besteht aus einem oder mehreren Reaktionsbereichen. Bei der Ausführungsform der Erfindung mit mehreren Reaktionsbereichen ist es vorteihaft, diese zu kaskadieren. Der flüssige Austragsstrom eines Reaktionsbereiches bildet dabei den Zulauf des nachfolgenden Reaktionsbereiches. Dies kann mit Hilfe eines Überlaufes geschehen. Für den Fall, daß es sich bei den einzelnen Reaktionsbereichen um voneinander getrennte Apparate handelt, ist deren Anzahl unter Berücksichtigung der Investitionskosten ≥ 2 und ≤ 4. Wird mehr als ein Reaktionsbereich innerhalb ein und desselben Reaktors geschaffen, (z.B. durch den Einsatz von Trennblechen), so kann die Anzahl der Reaktionsbereiche auch größer 4 sein. Im Falle von mehreren Reaktionsbereichen werden die Brüden der Reaktionsbereiche einer gemeinsamen Rektifikationskolonne zugeführt, deren flüssiger Ablauf vorteihafterweise in den ersten Reaktionsbereich gelangt. Es ist jedoch auch möglich und in speziellen Fällen auch angezeigt, auf mehrere Reaktionsbereiche je eine Rektifikationseinheit aufzusetzen und deren flüssigen Rücklauf in ein oder mehrere Reaktionsbereiche, vorteilhafterweise jeweils in den vorhergehenden, zurückzuführen.

Wird bei diesem Verfahren ein (Ausgangs)alkanol eingesetzt, das 4 - 8 C-Atome aufweist, beträgt die Temperatur im ersten Reaktionsbereich in der Regel 70 - 150°C, vorzugsweise 80 - 130°C, und im letzten Bereich 100 - 160°C, vorzugsweise 110 - 130°C. Die Reaktionstemperatur wird vorzugsweise so eingestellt, daß sie längs der Kaskade ansteigt. Der Druck in allen Reaktionsbereichen beträgt von 100 mbar bis Atmosphärendruck, vorzugsweise 200 mbar - 700 mbar. Der Druck ist in allen Reaktionsbereichen gleich. Die Gesamtverweilzeit der Reaktanden in den Reaktionsbereichen beträgt in der Regel 0.25 - 15 h, vorzugsweise 1 - 7 h, besonders bevorzugt 1,5 - 5 h. Vorzugsweise nimmt die Verweilzeit in aufeinander folgenden Reaktionsbereichen ab.

Bevorzugt wird als saurer Veresterungskatalysator para-Toluolsulfonsäure eingesetzt. Ihr Gehalt in der Reaktionszone bezogen auf das darin enthaltene Reaktionsgemisch beträgt zweckmäßigerweise 0,1 - 10 Gew.-%, bevorzugt 0,1 - 6 Gew.-%. Andere organische Sulfonsäuren wie Methansulfonsäure, Benzolsulfonsäure, Dodecylsulfonsäure und/oder Schwefelsäure sind ebenfalls einsetzbar. Deren Menge ist äquimolar zu der der para-Toluolsulfonsäure. Auch entsprechende Mischungen sind möglich. Der Gehalt an katalytisch wirksamer Säure im Sumpf der Rektifikationseinheit I bezogen auf das darin enthaltene Gemisch kann vorteilhafterweise zwischen 2,5 und 50 Gew.-% para-Toluolsulfonsäure betragen oder eine dazu äquimolaren Menge an anderer organischer Sulfonsäure und/oder Schwefelsäure.

In der Regel wird sowohl die (Meth)acrylsäure als auch der saure Veresterungskatalysator der Reaktionszone direkt zugeführt. Das zu veresternde Alkanol wird vorzugsweise der Reaktionszone über die auf diese aufgesetzte Rektifikationseinheit III zugeführt. Diese Rektifikationseinheit III kann aus einer Rektifikationskolonne bekannter Bauart beispielsweise mit Glockenböden oder Siebböden bestehen. Die Reaktionsbereiche können vorteilhafterweise aus Reaktoren mit Natur- oder Zwangsumlaufverdampfern bestehen.

Je nachdem, welches Alkanol verestert werden soll, sind in Einzelheiten voneinander abweichende Verfahrensweisen sinnvoll und zweckmäßig.

Die Veresterung von Alkanolen mit mehr als 4 C-Atomen, insbesondere mit 8 C-Atomen, bei denen die Siedepunkte des Esters und der (Meth)acrylsäure weiter auseinander liegen und daher in der Rektifikationseinheit I üblicherweise kein Wasser als Leichtsiederazeotrop bildender Hilfsstoff zugeführt werden muß, sollen anhand des Beispiels der Veresterung von 2-Ethylhexanol näher beschrieben werden.

Bei der Veresterung von 2-Ethylhexanol wird von der am Kopf der Rektifikationseinheit III anfallenden wäßrigen Phase ein Teil in diese Rektifikationseinheit zurückgeführt. Das aus der Reaktionszone ausgetragene Produktgemisch wird der Rektifikationseinheit I zugeführt. Das in die Rektifikationseinheit I geführte Produktgemisch wird in dieser in ein den 2-Ethylhexylester der (Meth)acrylsäure verbliebenes 2-Ethylhexanol und verbliebene (Meth)acrylsäure enthaltendes Produkt I und in ein den sauren Veresterungskatalysator und schwerer als den 2-Ethylhexylester der (Meth)acrylsäure siedende Komponenten enthaltendes Produkt II aufgetrennt. Dabei wird zweckmäßigerweise als Rektifikationseinheit I wieder eine Rektifikationskolonne I eingesetzt. Dieser wird das aus der Reaktionszone ausgetragene Produktgemisch üblicherweise im unteren Teil zugeführt. Das Produkt II wird normalerweise aus dem Sumpf dieser Rektifikationskolonne und das Produkt I normalerweise an deren Kopf gewonnen. Ein Teil des Produkts II wird zweckmäßigerweise in die Reaktionszone, vorzugsweise in den ersten Reaktionsbereich entweder direkt und/oder über die Rektifikationseinheit III zurückgeführt. Vorteilhafterweise wird ein Teil des Produkts II ausgetragen und einer Destillationseinheit IV zugeführt und in dieser aufgetrennt in ein 2-Ethylhexanol, (Meth)acrylsäure und 2-Ethylhexylester der (Meth)acrylsäure enthaltendes Produkt III und ein den sauren Veresterungskatalysator sowie höher als der 2-Ethylhexylester der (Meth)acrylsäure siedenden Komponenten enthaltendes Produkt IV.

Das Produkt III kann dann in die Rektifikationseinheit I und/oder in die Reaktionszone rückgeführt werden. Aus dem Produkt II und/oder dem Produkt IV können durch Extraktion mit Wasser saurer Veresterungskatalysator teilweise oder vollständig abgetrennt und die anfallende wäßrige Phase teilweise oder vollständig in die Reaktionszone zurückgeführt werden. Zu dieser Extraktion kann ein Teil der in der Rektifikationseinheit III anfallenden wäßrigen Phase verwendet werden. Das der Rektifikationseinheit I entnommene Produkt I kann der Rektifikationseinheit II zugeführt und in dieser aufgetrennt werden in ein Produkt V, das verbliebenes 2-Ethylhexanol, (Meth)acrylsäure und leichter als 2-Ethylhexyl(meth)acrylat siedende Komponenten enthält, den Zielester 2-Ethylhexyl(meth)acrylat und ein Produkt VI, das schwerer siedende als 2-Ethylhexyl(meth)acrylat enthaltene Bestandteile aufweist. Das Produkt V kann dann in die Reaktionszone zurückgeführt werden, vorzugsweise in den zweiten Reaktionsbereich, das Produkt VI kann in die Rektifikationseinheit I zurückgeführt werden. Die Rektifikationseinheit II ist zweckmäßigerweise als Rektifikationskolonne ausgebildet. Hier kann das Produkt V im oberen Teil der Rektifikationskolonne II, das Produkt VI aus deren Sumpf und das 2-Ethylhexyl(meth)acrylat als dampfförmiger Seitenabzug im unteren Teil abgetrennt werden.

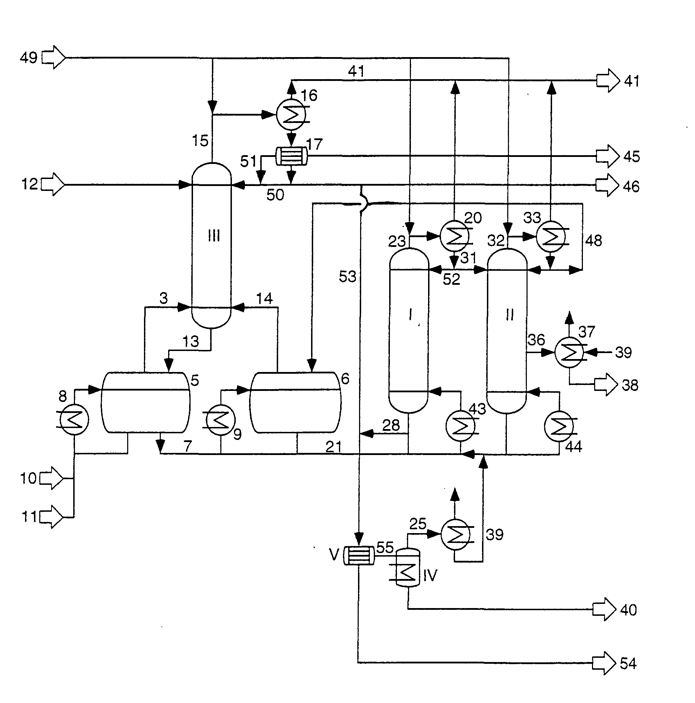

Weitere Einzelheiten und Vorteile der Erfindung können dem anhand der Zeichnung beschriebenen Ausführungsbeispiel entnommen werden. Die Zeichnung zeigt
in Fig. 1 im Verfahrensschema eine Anlage zur Herstellung von 2-Ethylhexylacrylat.

Die Rektifikationskolonnen sind mit römischen Bezugszeichen versehen. Der Übersichtlichkeit wegen sind außerdem die allgemein mit römischen Ziffern versehenen Produktbezeichnungen in diesem speziellen Ausführungsbeispiel konkret eingefügt.

Die dargestellte Anlage hat drei Rektifikationskolonnen I, II, III und einen Rührkessel IV. Sie ist außerdem mit zwei Veresterungsreaktoren 5 und 6 versehen, die über eine Leitung 7 hintereinander geschaltet sind und so eine Reaktionskaskade bilden. An die Reaktoren 5 und 6 sind Umlaufverdampfer 8 und 9 angeschlossen.

Die Veresterungsreaktion durch Umsetzung von 2-Ethylhexanol und Acrylsäure erfolgte in der zweistufigen Veresterungskaskade, die aus den beiden gleichvolumigen Reaktoren 5 und 6 bestand. Dem Reaktor 5 wurden über eine Leitung 10 Acrylsäure und über eine Leitung 11 p-Toluolsulfonsäure als Katalysator über den Umlaufverdampfer 8 zugeführt. Die Reaktionskomponente 2-Ethylhexanol wurde über eine Leitung 12 auf den Kopf einer Destillationskolonne III aufgegeben, deren unteres Ende über eine Leitung 13 mit dem Reaktor 5 verbunden war. Der bei der Veresterung in den Reaktoren 5 und 6 gebildete, das Reaktionswasser enthaltende Dampf wurde über Leitungen 3 und 14 der Destillationskolonne III zugeführt, die 20 Glockenböden aufwies und bei einem Druck von 270 mbar betrieben wurde. Dieser Druck wurde durch eine Leitung 15, die zu einem Kühler 16 führte, über eine zu einer Vakuumpumpe führenden Leitung 41 aufrechterhalten. Das im Kühler 16 gebildete Kondensat wurde im Abscheider 17 in zwei flüssige Phasen aufgetrennt. Abgetrennte Oktene wurden durch Leitung 45 abgeführt. Reaktionswasser wurde durch die Leitung 46 abgeführt.

Da die Edukte dem Reaktor 5 mit Umgebungstemperatur zugeführt wurden und bei der Reaktion eine hohe Wassermenge gebildet wird, ist für die Aufrechterhaltung einer Reaktionstemperatur von 110 °C ein hoher Wärmeeintrag in den ersten Reaktor 5 erforderlich. Dies macht den Einsatz eines außenliegenden Umlaufverdampfers 8 möglich. Das aus dem Reaktor 5 über Leitung 7 ausgetragene Sumpfprodukt wurde über einen weiteren Umlaufverdampfer 9 dem zweiten Reaktor 6 zugeführt, in dem eine Temperatur von 120 °C eingestellt wurde. Der zweite Verdampfer 9 wurde als Umlaufverdampfer ausgeführt, um trotz wesentlich geringeren Wärmeeintrags eine für die Durchmischung des Kesselinhalts ausreichende Umlaufmenge sicherzustellen. Auch hier wurde Magerluft als Co-Stabilisator zugegeben. Der abnehmenden Acrylsäure- und Wasser-Konzentration angepaßt wurde der zweite Reaktor 6 bei erhöhter Temperatur betrieben. Das im Reaktor 6 entstehende Sumpfprodukt wurde durch Leitung 21 ausgetragen. Der gesamte Reaktionsaustrag, der das Zielprodukt, d.h. das gebildete 2-Ethylhexylacrylat sowie alle leichter siedenden Edukte und Nebenprodukte enthält, wurde über Leitung 21 und einen weiteren Umlaufverdampfer 43 in den unteren Teil einer Rektifikationskolonne I eingeleitet, die als eine Verstärkungskolonne ausgebildet war und mit zehn Dual-Flow-Böden der Schwersiederabtrennung diente. Das Zielprodukt, nämlich 2-Ethylhexylacrylat, sowie alle leichtersiedenden Edukte und Nebenprodukte wurden über Kopf durch Leitung 23 abgeführt und nach Durchströmen eines mit der Vakuumleitung 41 verbundenen Kühlers über Leitung 31 dem Kopf einer als eine Abtriebssäule betriebenen Reinkolonne II zugeführt.

Die Kolonne I für die Schwersiederabtrennung wurde bei einem Sumpfdruck von 100 mbar und einem Kopfdruck von 70 mbar betrieben. Die Temperatur betrug 150 °C.

Der in der Kolonne I für die Schwersiederabtrennung anfallende Sumpf wurde über eine Leitung 28 ausgetragen, auf 50°C abgekühlt und einer Extraktionseinheit (V) zugeführt. Unter Zugabe eines Teiles des Veresterungswassers durch Leitung 53 wurde der Gehalt an Paratoluolsulfonsäure der organischen Phase auf für die Spaltung optimale 1,5 % abgesenkt. Der mit bis zu 30 % p-Toluolsulfonsäure beladene, ablaufende Wasserstrom wurde durch Leitung 54 abgezogen, die organische Phase durch Leitung 55 und einer Destillationseinheit IV zugeführt. In dieser wurde bei einer Temperatur von 180 °C und einem Druck von 60 mbar diskontinuierlich zunächst noch enthaltenes Produkt abgedampft. Anschließend wurde der hoch p-Toluolsulfonsäure und Oxyester-haltige Rückstand in Edukte, Zielprodukt, Wasser und die als Nebenprodukt anfallenden Oktene gespalten. Das vereinigte Kopfprodukt aus der Spaltung wurde über Leitung 25 abgezogen, verflüssigt und über Leitung 39 in den Sumpf der Schwersiederabtrennung zurückgeführt. Der verbleibende zähflüssige Rückstand wurde durch Leitung 40 abgezogen und in einer Rückstandsverbrennung entsorgt.

Aus dem der Reinkolonne II durch Leitung 31 über Kopf zugeführten Produkt wurden die noch vorhandenen Edukte und leichtersiedenden Nebenkomponenten über Leitung 32 abgezogen und einem Kondensator 33 zugeführt. Das dabei anfallende Kondensat wurde über Leitung 48 in die zweite Stufe 6 der Veresterungskaskade zurückgeführt. Der Sumpf der Kolonne II wurde durch einen, den in der Veresterung eingesetzten Apparaten analogen Umlaufverdampfer 44 beheizt.

Das Reinprodukt 2-Ethylhexylacrylat wurde über Leitung 36 dampfförmig abgezogen und über einen berieselten Demister dem Kondensator 37 zugeführt, um Farbzahlprobleme zu vermeiden und das Umstabilisieren des Produkts von Phenothiazin als Prozeßinhibitor auf Hydrochinonmonomethylether als Lagerstabilisator zu ermöglichen. Das Reinprodukt wurde durch Leitung 38 abgeführt. Der Lagerstabilisator wurde durch Leitung 39 zugeführt. Das als Prozeßinhibitor eingesetzte Phenothiazin wurde über Leitung 49 den Rektifikationskolonnen I, II, III über Kopf zugeführt.

Im folgenden soll nun ein spezielles Ausführungsbeispiel beschrieben werden, das mit einer Versuchsapparatur durchgeführt worden ist, wie sie in der Zeichnung dargestellt ist. Dabei wurden zwei Veresterungskessel 5 und 6 mit je 2 l Nutzvolumen eingesetzt, auf die eine Glasbodenkolonne mit einem Durchmesser von 50 mm aufgesetzt war, die mit 20 Glockenböden sowie einem Phasenscheider am Kolonnenkopf ausgestattet war. Der Betriebsdruck betrug 270 mbar. Zur Beheizung der Veresterungskessel wurden Umlaufverdampfer eingesetzt. Bei einer Verweilzeit von 4 Stunden wurde Acrylsäure mit 2-Ethylhexanol im stöchiometrischen Verhältnis unter Zugabe von 1,5 Gew.-% wäßriger p-Toluolsulfonsäure-Lösung unter kontinuierlicher und destillativer Abtrennung des gebildeten Reaktionswassers zu 2-Ethylhexylacrylat (EHA) umgesetzt. Die Temperatur im ersten Veresterungskessel 5 betrug 110 °C, im zweiten Veresterungskessel 6 betrug die Temperatur 120 °C. Im Austrag des ersten Reaktors 5 wurde eine Konzentration an EHA von 70 Gew.-% und im Austrag des zweiten Reaktors 6 von 82 Gew.-% erreicht. Leichtsiedende Nebenkomponenten (in der Hauptsache in der Spaltung gebildete Oktene) wurden im Kopf der Veresterungskolonne III soweit konzentriert, daß der über Leitung 45 abgezogene Ausschleusstrom nur noch < 10% Wertkomponenten, d.h. 2-Ethylhexanol und 2-Ethylhexylacrylat, enthielt. Durch wäßrigen Kolonnenrücklauf wurden über die gesamte Kolonnenhöhe zwei flüssige Phasen eingestellt. Dadurch konnte die Acrylsäure im Kolonnenkopf auf < 100 ppm abgereichert werden. Das in stöchiometrischer Menge anfallende Veresterungswasser war zu etwa 1,5% mit organischen Verbindungen (in der Hauptsache 2-Ethylhexanol und Oktenen) im Gleichgewicht beladen.

Der Veresterungsaustrag durch Leitung 21 wurde in einer mit Umlaufverdampfer und Wärmeübertrager ausgestatteten Laborkolonne I mit 50 mm Durchmesser und 10 Dual-Flow-Böden von der Katalysatorsäure und den gebildeten Schwersiedern befreit. 5% der zulaufenden Rohestermenge wurden bei einem Rücklaufverhältnis von 0,5 aus dem Sumpf der Kolonne als Schwersiederaustrag über eine Extraktionsstufe in die Spaltung abgezogen, der Rest als schwersiederfreies (Oxyester < 10 ppm) Kopfprodukt. Der Zulauf erfolgte unmittelbar in den Kolonnensumpf und die Kolonne I wurde als reine Verstärkungssäule betrieben. Bei einem Kopfdruck von 80 mbar konnte eine maximale Sumpftemperatur von 150 °C eingehalten werden. Das schwersiederfreie Kopfprodukt wurde in einer mit 25 Dual-Flow-Böden ausgestatteten Laborkolonne II von 50 mm Durchmesser bei einem Kopfdruck von 80 mbar und einer maximalen Sumpftemperatur von 140 °C in eine die Edukte Acrylsäure und 2-Ethylhexanol sowie 50 Gew.-% 2-Ethylhexylacrylat enthaltende Kopffraktion und das Reinprodukt aufgetrennt. Die Kopffraktion wurde in den zweiten Veresterungsreaktor 6 zurückgeführt. Das Reinprodukt wurde frei von Schwersiedern und Prozeßstabilisatoren dampfförmig aus dem Kolonnensumpf, der mit einem Umlaufverdampfer beheizt wurde, abgezogen und in einem durch Inertenüberdeckung geregelten Kondensator verflüssigt. Dabei wurde ein Gehalt von > 99,8 Gew.-% 2-Ethylhexylacrylat erreicht. Eine Aufpegelung schwersiedender Spurenkomponenten im Kolonnensumpf wurde durch einen flüssigen Sumpfabzug in den Sumpf der Schwersiederabtrennung von 2% der zur Laborkolonne II zulaufenden Flüssigkeitsmenge verhindert. Der Sumpfaustrag der Schwersiederabtrennung wurde nach teilweiser Extraktion der Katalysatorsäure mit Wasser in einem diskontinuierlich betriebenen Spaltkessel IV bei 60 mbar und einer Maximaltemperatur von 180 °C auf 20% seiner ursprünglichen Masse eingeengt. Der entstehende Rückstand enthielt neben der Katalysatorsäure p-Toluolsulfonsäure nicht spalt- und verdampfbare Schwersieder in hoher Konzentration. Dieser Rückstand war nicht weiter im Prozeß verwertbar und wurde abgezogen. Das zu 80% aus EHA, 10 bis 12% Oktenen sowie aus Acrylsäure, Wasser und 2-Ethylhexanol bestehende Kopfprodukt wurde in einem Wärmeaustauscher niedergeschlagen und in den Sumpf der Schwersiederabtrennung rückgeführt.

Im kontinuierlichen, stationären Betrieb dieser Versuchsanlage konnte eine Eduktausbeute von 98% erreicht werden. Nur 2% der eingesetzten Edukte gingen als Nebenprodukte verloren.

Als Stabilisatorlösung diente eine 2%ige Phenothiazinlösung in 2-Ethylhexanol, die jeweils in die Kopfkondensatoren der einzelnen Verfahrensstufen in einer Aufwandmenge von 100 ppm, bezogen auf den jeweiligen Zulaufstrom der Stufe, entsprechend dosiert wurde. Alle Naturumlaufverdampfer wurden mit Luft als Co-Stabilisator beaufschlagt.

Besonders vorteilhaft bei dem vorstehend beschriebenen Verfahren ist die Abtrennung aller schwersiedenden Nebenkomponenten und insbesondere des Katalysators aus dem Veresterungsaustrag in Kolonne I. Dadurch wird eine Rückspaltung der Schwersieder und/oder des Zielproduktes im Sumpf der Reinkolonne II in Edukte und damit eine Verunreinigung des Reinproduktes mit leichtsiedenden Spaltprodukten und insbesondere Acrylsäure sicher vermieden.

Werden, wie bei konventionellen Verfahrensweisen üblich, aus dem Veresterungsaustrag zunächst die leichter als der Zielester siedenden Komponenten (insbesondere Acrylsäure und Alkanol) abgetrennt, so ist es aufgrund der dann im Sumpf der Reinkolonne in Anwesenheit von Katalysator und Schwersiedern einsetzenden Spaltreaktionen nicht möglich, leichtsieder- und insbesondere acrylsäurefreies Reinprodukt zu gewinnen, wie durch das nachfolgend beschriebene Ausführungsbeispiel nachgewiesen wurde:

Aus Leitung 21 entnommener Veresterungsaustrag wurde zunächst in Kolonne I von allen leichter als der Zielester siedenden Nebenkomponenten sowie den Edukten Acrylsäure und 2-Ethylhexanol befreit. Aus dem Sumpf der Kolonne I wurde dann der leichtsiederfreie, aber mit Schwersiedern und insbesondere dem Katalysator verunreinigte Rohester entnommen und in einer mit 25 Dual-Flow-Böden ausgestatteten, 50 mm durchmessenden Laborkolonne bei einem Rücklaufverhältnis von 2 rektifiziert. Der dabei im Kopf der Kolonne entnommene Rohester war mit 1400 ppm Acrylsäure verunreinigt, obwohl der zulaufende Rohester acrylsäurefrei war; d.h. die im Zielester nachgewiesene Acrylsäure kann nur durch Spaltreaktionen im Sumpf der Kolonne entstanden sein. Eine destillative Abtrennung der Acrylsäure ist jedoch bei der Gewinnung des Reinesters als Kopfprodukt nicht möglich, da Acrylsäure gegenüber dem Zielester einen Leichtsieder darstellt.

Den vorstehend beschriebenen Versuchsergebnissen sowie weiteren Untersuchungen konnte entnommen werden, daß es zweckmäßig ist, die beiden Veresterungsreaktoren 5 und 6 bei Drücken zwischen 180 bis 500 mbar, vorzugsweise 180 bis 350 mbar, zu betreiben. Dabei kann die Temperatur im ersten Reaktor 5 bei 80 bis 120 °C, im zweiten Reaktor 6 bei 100 bis 140 °C liegen. Als Katalysator für die Veresterungsreaktion in den Reaktoren 5 und 6 haben sich saure Katalysatoren, insbesondere organische Sulfonsäuren und hier besonders p-Toluolsulfonsäure in einer Menge von 0,1 bis 4, vorzugsweise 0,5 bis 2 Gew.-%, als besonders vorteilhaft erwiesen.

Die Verweilzeit von (Meth)acrylsäure und Alkanol in den Reaktoren liegt zwischen 0,5 und 8 Stunden, vorzugsweise zwischen 1 und 6 Stunden. Von der durch Leitung 21 der Rektifikationskolonne I zugeführten Rohestermenge kann bei einem Rücklaufverhältnis von 0,5 eine Menge < 10, vorzugsweise < 5 Gew.-%, aus dem Sumpf der Kolonne als Schwersiederabtrennung über eine Extraktion in die Spaltung abgezogen werden. Der Kopfdruck der Kolonne I kann 50 bis 400 mbar, vorzugsweise < 120 mbar, betragen. Die maximale Sumpftemperatur dieser Kolonne beträgt vorzugsweise < 150 °C. Das schwersiederfreie Kopfprodukt der Kolonne I, das über die Leitung 31 der Kolonne II zugeführt wurde, kann dort bei einem Kopfdruck von 50 bis 400 mbar, vorzugsweise bei einem Druck < 120 mbar, und bei einer Sumpftemperatur < 140 °C bearbeitet werden. Bei optimaler Einstellung der vorgenannten Werte kann durch Leitung 36 ein Produkt abgezogen werden, dessen Gehalt an Reinprodukt, im Falle des Ausführungsbeispiels an 2-Ethylhexylacrylat, > 99,8 Gew.-% beträgt.

## Patentansprüche

1. Verfahren zur kontinuierlichen Herstellung von Alkylestern der (Meth)acrylsäure durch Umsetzung von (Meth)acrylsäure mit 4 bis 8 C-Atome aufweisenden Alkanolen in homogener, flüssiger, an Lösungsmittel freier Phase bei erhöhter Temperatur und in Gegenwart eines sauren Veresterungskatalysators, bei dem man die (Meth)acrylsäure, das Alkanol und den Katalysator einer Reaktionszone (5, 6) zuführt, während einer Verweilzeit das gebildete Wasser als Bestandteil eines Alkanol umfassenden Gemisches in einer der Reaktionszone (5, 6) aufgesetzten Rektifikationseinheit (III) rektifikativ abtrennt, das dabei anfallende Destillat in eine Alkanol enthaltende organische und in eine Wasser enthaltende wäßrige Phase auftrennt, die organische Phase *mit Ausnahme eines Teilstroms, der ausgeschleust wird* in die Rektifikationseinheit (III) zurückführt, das Reaktionsgemisch aus der Reaktionszone (5, 6) austrägt und in eine weitere Rektifikationseinheiten (I, II) umfassende destillative Trennzone leitet und in dieser den gebildeten Alkylester der (Meth)acrylsäure abtrennt. dadurch gekennzeichnet, daß
a) (Meth)acrylsäure und Alkanol im Molverhältnis von 1:0,75 bis 1:2 umgesetzt werden,
b) von der am Kopf der Rektifikationseinheit (III) anfallenden wäßrigen Phase ein Teil in die Rektifikationseinheit zurückgeführt wird,
c) das aus der Reaktionszone ausgetragene Reaktionsgemisch einer weiteren Rektifikationseinheit (I) zugeführt und in dieser das Reaktionsgemisch in ein den Katalysator enthaltendes Produkt (II) und in ein den Alkylester der (Meth)acrylsäure, verbliebenes Alkanol und verbliebene (Meth)acrylsäure enthaltendes Produkt (I) aufgetrennt wird, und
d) das Produkt (I) einer weiteren Rektifikationseinheit (II) zugeführt und in dieser der Alkylester der (Meht)acrylsäure vom verbliebenen Alkanol und von der verbliebenen (Meth)acrylsäure abgetrennt und das verbliebene Alkanol und die verbliebene (Meth)acrylsäure in die Reaktionszone zurückgeführt werden.

2. Verfahren nach Anspruch 1, dadurch gekennzeichnet, daß die Reaktionszone aus einer Kaskade von mindestens zwei hintereinander geschalteten Reaktionsbereichen besteht und der Austragsstrom eines Reaktionsbereichs einen Zulaufstrom eines nachfolgenden Reaktionsbereichs bildet, wobei die Kaskade insbesondere 2 bis 4 voneinander räumlich getrennte Reaktionsbereiche aufweist.

3. Verfahren nach Anspruch 2, dadurch gekennzeichnet, daß die Temperatur im ersten Reaktionsbereich 70 bis 150°C und im letzten Bereich 100 bis 160°C beträgt.

4. Verfahren nach Anspruch 2 oder 3, dadurch gekennzeichnet, daß der Druck in allen Reaktionsbereichen von 100 mbar bis Atmosphärendruck beträgt und in allen Reaktionsbereichen gleich ist.

5. Verfahren nach einem der Ansprüche 1 bis 4, dadurch gekennzeichnet, daß die Gesamtverweilzeit der Reaktanden in den Reaktionsbereichen 0,25 bis 15 h beträgt.

6. Verfahren nach einem der Ansprüche 1 bis 5, dadurch gekennzeichnet, daß die aufsteigenden Dämpfe aus den Reaktionsbereichen der Rektifikationseinheit (III) zugeführt werden, deren flüssiger Rücklauf nur in den ersten Reaktionsbereich zurückgeführt wird.

7. Verfahren nach einem der Ansprüche 1 bis 6, dadurch gekennzeichnet, daß als Katalysator para-Toluolsulfonsäure und/oder andere organische Sulfonsäuren wie Methansulfonsäure, Benzolsulfonsäure, Dodecylbenzolsulfonsäure und/oder Schwefelsäure eingesetzt werden, wobei der Gehalt an katalytisch wirksamer Säure in der Reaktionszone bezogen auf das darin enthaltene Reaktionsgemisch 0,1 bis 10 Gew.-% an para-Toluolsulfonsäure beträgt oder einer dazu äquimolaren Menge an anderer organischer Sulfonsäure und/oder Schwefelsäure und der Gehalt an katalytisch wirksamer Säure in Sumpf der Rektifikationseinheit (I) bezogen auf das darin enthaltene Gemisch zwischen 2,5 und 50 Gew.-% para-Toluolsulfonsäure beträgt oder einer dazu äquimolaren Menge an organischer Sulfonsäure und/oder Schwefelsäure.

8. Verfahren nach einem der Ansprüche 1 bis 7, dadurch gekennzeichnet, daß das zu veresternde Alkanol 2-Ehtylhexanol ist, sowohl die (Meth)acrylsäure als auch der Katalysator der Reaktionszone direkt zugeführt werden und das zu veresternde Alkanol der Reaktionszone über die Rektifikationseinheit (III) zugeführt wird.

9. Verfahren nach einem der Ansprüche 1 bis 8, dadurch gekennzeichnet, daß die Rektifikationseinheit (III) aus einer Rektifikationskolonne besteht und die Reaktionsbereiche aus Reaktoren mit Umlaufverdampfern bestehen.

10. Verfahren nach Anspruch 8, dadurch gekennzeichnet, daß das in die Rektifikationseinheit (I) zugeführte Produktgemisch in der Rektifikationseinheit (I) in ein den 2-Ethylhexylester der (Meth)acrylsäure, verbliebenes 2-Ethylhexanol und verbliebene (Meth)acrylsäure enthaltendes Produkt (I) und in ein den Katalysator und schwerer als der 2-Ethylhexylester der (Meth)acrylsäure siedende Komponenten enthaltendes Produkt (II) aufgetrennt wird.

11. Verfahren nach Anspruch 10, dadurch gekennzeichnet, daß die Rektifikationseinheit (I) eine Rektifikationskolonne (I) ist, das aus der Reaktionszone ausgetragene Produktgemisch dem unteren Teil der Rektifikationskolonne (I) zugeführt wird, das Produkt (II) aus dem Sumpf der Rektifikationskolonne (I) und das Produkt (I) am Kopf der Rektifikationskolonne (I) gewonnen werden und ein Teil des Produktes (II) in die Reaktionszone, in den ersten Reaktionsbereich, entweder direkt und/oder über die Rektifikationseinheit (III) zurückgeführt wird.

12. Verfahren nach Anspruch 11, dadurch gekennzeichnet, daß ein Teil des Produktes (II) ausgetragen und einer weiteren Destillationseinheit (IV) zugeführt und in dieser in ein 2-Ethylhexanol, (Meth)acrylsäure und 2-Ethylhexylester der (Meth)acrylsäure enthaltendes Produkt (III) und ein den Katalysator sowie höher als der 2-Ethylhexylester der (Meth)acrylsäure siedende Komponenten enthaltendes Produkt (IV) aufgetrennt wird, das Produkt (III) in die Rektifikationseinheit (I) und/oder in die Reaktionszone zurückgeführt wird, aus dem Produkt (II) und/oder dem Produkt (IV) durch Extraktion mit Wasser Katalysator abgetrennt und die anfallende wäßrige Phase in die Reaktionszone zurückgeführt wird und zur Extraktion ein Teil der in der Rektifikationseinheit (III) anfallenden wäßrigen Phase verwendet wird.

13. Verfahren nach Anspruch 12, dadurch gekennzeichnet, daß das der Rektifikationseinheit (I) entnommene Produkt (I) einer Rektifikationseinheit (II) zugeführt und in dieser aufgetrennt wird in ein verbliebenes 2-Ethylhexanol, (Meth)acrylsäure und leichter als 2-Ethylhexyl(meth)acrylat siedende Komponenten enthaltendes Produkt (V), 2-Ethylhexyl(meth)acrylat und ein schwerer als 2-Ethyl(meth)acrylat siedendes Produkt (VI).

14. Verfahren nach Anspruch 13, dadurch gekennzeichnet, daß das Produkt (V) in die Reaktionszone zurückgeführt wird, das Produkt (VI) in die Rektifikationseinheit (I) zurückgeführt wird, die Rektifikationseinheit (II) eine Rektifikationskolonne (II), und das Produkt V im oberen Teil der Rektifikationskolonne (II), das Produkt (VI) aus dem Sumpf der Rektifikationskolonne (II) und das 2-Ethylhexyl(meth)acrylat als dampfförmiger Seitenabzug im unteren Teil der Rektifikationskolonne (II) abgetrennt werden.

## Claims

1. A process for the continuous preparation of alkyl esters of (meth)acrylic acid by reacting (meth)acrylic acid and alkanols having from 4 to 8 carbon atoms in a homogeneous, liquid, solvent-free phase at elevated temperature and in the presence of an acid esterification catalyst, in which the (meth)acrylic acid, the alkanol and the catalyst are fed to a reaction zone (5,6), the water formed is removed by rectification during a residence time as constituent of a mixture comprising alkanol in a rectification unit (III) superposed on the reaction zone (5,6), the distillate obtained is separated into an organic phase comprising alkanol and an aqueous phase comprising water, the organic phase, with the exception of a substream which is bled off, is returned to the rectification unit (III), the reaction mixture is discharged from the reaction zone (5,6) and conveyed into a distillative separation zone comprising further rectification units (I, II) and in the latter the alkyl (meth)acrylate formed is separated off,
wherein
a) (meth)acrylic acid and alkanol are reacted in a molar ratio of from 1:0.75 to 1:2,
b) part of the aqueous phase obtained at the top of the rectification unit (III) is returned to the rectification unit,
c) the reaction mixture discharged from the reaction zone is fed to a further rectification unit (I) and in this the reaction mixture is separated into a product (II) comprising the catalyst and a product (I) comprising the alkyl ester of (meth)acrylic acid, remaining alkanol and remaining (meth)acrylic acid,
d) the product (I) is fed to a further rectification unit (II) and in this the alkyl ester of (meth)acrylic acid is separated from the remaining alkanol and from the remaining (meth)acrylic acid and the remaining alkanol and the remaining (meth)acrylic acid are returned to the reaction zone.

2. A process as claimed in claim 1, wherein the reaction zone comprises a cascade of at least two reaction regions connected in series and the product stream of one reaction region forms a feed stream of a downstream reaction region, where, in particular, the cascade comprises from 2 to 4 reaction regions separated in space from one another.

3. A process as claimed in claim 2, wherein the temperature in the first reaction region is 70 - 150°C, and in the last region is 100 - 160°C.

4. A process as claimed in claim 2 or 3, wherein the pressure in all reaction regions is from 100 mbar to atmospheric pressure and is the same in all reaction regions.

5. A process as claimed in any of claims 1 to 4, wherein the total residence time of the reactants in the reaction regions is from 0.25 to 15 hours.

6. A process as claimed in any of claims 1 to 5, wherein the rising vapors from the reaction regions are fed to the rectification unit (III) whose liquid runback is returned only to the first reaction region.

7. A process as claimed in any of claims 1 to 6, wherein the esterification catalyst used is para-toluenesulfonic acid and/or other organic sulfonic acids such as methanesulfonic acid, benzenesulfonic acid, dodecylbenzenesulfonic acid and/or sulfuric acid and the content of catalytically active acid in the reaction zone, based on the reaction mixture present therein, is from 0.1 to 10 % by weight of para-toluenesulfonic acid, or an amount equimolar thereto of another organic sulfonic acid and/or sulfuric acid and the content of catalytically active acid in the liquid phase of the rectification unit (I), based on the mixture present therein, is from 2.5 to 50 % by weight of para-toluenesulfonic acid or an amount equimolar thereto of organic sulfonic acid and/or sulfuric acid.

8. A process as claimed in any of claims 1 to 7, wherein the alkanol to be esterified is 2-ethylhexanol, both the (meth)acrylic acid and the catalyst are fed directly to the reaction zone, and the alkanol to be esterified is fed to the reaction zone via the rectification unit (III).

9. A process as claimed in any of claims 1 to 8, wherein the rectification unit (III) is a rectification column, and the reaction regions comprise reactors with convection vaporizers.

10. A process as claimed in claim 8, wherein the product mixture fed to the rectification unit (I) is, in the rectification unit (I), separated into a product (I) comprising the 2-ethylhexyl ester of (meth)acrylic acid, remaining 2-ethylhexanol and remaining (meth)acrylic acid and into a product (II) comprising the catalyst and components having boiling points higher than that of the 2-ethylhexyl ester of (meth)acrylic acid.

11. A process as claimed in claim 10, wherein the rectification unit (I) is a rectification column (II), the product mixture discharged from the reaction zone is fed to the lower part of the rectification column (I), the product (II) is obtained at the bottom of the rectification column (I) and the product (I) is obtained at the top of the rectification column (I), and part of the product (II) is returned to the reaction zone, in the first reaction region, either directly and/or via the rectification unit (III).

12. A process as claimed in claim 11, wherein part of the product (II) is discharged and fed to a further distillation unit (IV) and in this is separated into a product (III) comprising 2-ethylhexanol, (meth)acrylic acid and the 2-ethylhexyl ester of (meth)acrylic acid and a product (IV) comprising the catalyst and components having boiling points higher than that of the 2-ethylhexyl ester of (meth)acrylic acid, the product (III) is returned to the rectification unit (I) and/or the reaction zone, catalyst is separated from the product (II) and/or the product (IV) by extraction with water and the aqueous phase obtained is returned to the reaction zone, and the extraction is carried out using part of the aqueous phase obtained in the rectification unit (III).

13. A process as claimed in claim 12, wherein the product (I) taken from the rectification unit (I) is fed to a rectification unit (II) and in this is separated into a product (V) comprising remaining 2-ethylhexanol, (meth)acrylic acid and components having boiling points lower than that of 2-ethylhexyl (meth)acrylate, 2-ethylhexyl (meth)acrylate and a product (VI) having a boiling point higher than that of 2-ethylhexyl (meth)acrylate.

14. A process as claimed in claim 13, wherein the product (V) is returned to the reaction zone, the product (VI) is returned to the rectification unit (I), the rectification unit (II) is a rectification column (II), the product (V) is separated off in the upper part of the rectification column (II), the product (VI) is taken from the bottom of the rectification column (II) and the 2-ethylhexyl (meth)acrylate is taken off in vapor form at the side in the lower part of the rectification column (II).

## Revendications

1. Procédé pour préparer en continu des esters alkyliques de l'acide (méth)acrylique par réaction de l'acide (méth)acrylique avec des alcanols comportant 4 à 8 atomes de C dans une phase homogène, liquide, exempte de solvant, à une température élevée et en présence d'un catalyseur d'estérification acide, procédé dans lequel l'on introduit l'acide (méth)acrylique, l'alcanol et le catalyseur dans une zone de réaction (5, 6), l'on sépare par rectification, pendant une certaine durée de séjour, l'eau formée en tant que composant d'un mélange comprenant un alcanol, dans une unité de rectification (III) montée dans la zone de réaction (5, 6), l'on sépare le distillat ainsi produit en une phase aqueuse contenant de l'eau et en une phase organique contenant un alcanol, l'on recycle la phase organique dans l'unité de rectification (III) *à l'exception d'un courant partiel qui est évacué*, l'on soutire le mélange réactionnel de la zone de réaction (5, 6) et on le conduit dans une autre zone de séparation par distillation, comprenant d'autres unités de rectification (I, II) et on y sépare l'ester alkylique de l'acide (méth)acrylique formé, procédé caractérisé en ce que,
a) l'acide (méth)acrylique et l'alcanol sont convertis dans un rapport molaire de 1:0,75 à 1:2,
b) l'on recycle une partie de la phase aqueuse produite à la tête de l'unité de rectification (III), dans l'unité de rectification,
c) l'on introduit le mélange réactionnel extrait de la zone de réaction dans une autre unité de rectification (I) et dans celle-ci, l'on sépare le mélange réactionnel en un produit (II) contenant le catalyseur et en un produit (I) contenant l'ester alkylique de l'acide (méth)acrylique, l'alcanol restant et l'acide (méth)acrylique restant, et
d) l'on introduit le produit (I) dans une autre unité de rectification (II) et dans celle-ci, l'on sépare l'ester alkylique de l'acide (méth)acrylique d'avec l'alcanol restant et d'avec l'acide (méth)acrylique restant et l'on recycle l'alcanol restant et l'acide (méth)acrylique restant dans la zone de réaction.

2. Procédé selon la revendication 1, caractérisé en ce que la zone de réaction est constituée d'une cascade d'au moins deux secteurs de réaction placées l'un derrière l'autre et que le courant extrait d'un secteur de réaction forme un courant d'alimentation d'un secteur de réaction suivant, la cascade comprenant en particulier 2 à 4 secteurs de réaction spatialement séparés les uns des autres.

3. Procédé selon la revendication 2, caractérisé en ce que la température est de 70°C à 150°C dans le premier secteur de réaction, et de 100°C à 160°C dans le dernier secteur.

4. Procédé selon la revendication 2 ou 3, caractérisé en ce que la pression dans toutes les secteurs de réaction va de 100 mbars jusqu'à la pression atmosphérique, et en ce qu'elle est identique dans tous les secteurs de réaction.

5. Procédé selon l'une quelconque des revendications 1 à 4, caractérisé en ce que la durée totale de séjour des réactifs dans les secteurs de réaction est de 0,25 à 15 h.

6. Procédé selon l'une quelconque des revendications 1 à 5, caractérisé en ce que les vapeurs montantes provenant des secteurs de réaction sont introduites dans l'unité de rectification (III), dont le reflux liquide n'est recyclé que dans le premier secteur de réaction.

7. Procédé selon l'une quelconque des revendications 1 à 6, caractérisé en ce que l'on met en oeuvre en tant que catalyseur, l'acide para-toluène-sulfonique et/ou d'autres acides sulfoniques organiques comme l'acide méthane-sulfonique, l'acide benzène-sulfonique, l'acide dodécyl-benzène-sulfonique et/ou l'acide sulfurique, dans lequel la teneur en acide à action catalytique dans la zone de réaction est de 0,1 à 10% en poids d'acide para-toluène-sulfonique ou d'une quantité équimolaire d'un autre acide sulfonique organique et/ou d'acide sulfurique par rapport au mélange réactionnel qui y est contenu et la teneur en acide à action catalytique dans le fond de l'unité de rectification (I) est comprise entre 2,5 et 50% en poids d'acide para-toluène-sulfonique ou d'une quantité équimolaire d'un acide sulfonique organique et/ou d'acide sulfurique, par rapport au mélange qui y est contenu.

8. Procédé selon l'une quelconque des revendications 1 à 7, caractérisé en ce que l'alcanol à estérifier est le 2-éthylhexanol, que l'acide (méth)acrylique et le catalyseur sont tout deux introduits directement dans la zone de réaction et que l'alcanol à estérifier est introduit dans la zone de réaction par l'intermédiaire de l'unité de rectification (III).

9. Procédé selon l'une quelconque des revendications 1 à 8, caractérisé en ce que l'unité de rectification (III) se compose d'une colonne de rectification et les secteurs de réaction sont constituées de réacteurs ayant des évaporateurs à circulation.

10. Procédé selon la revendication 8, caractérisé en ce que le mélange de produits introduit dans l'unité de rectification (I) est séparé dans l'unité de rectification (I) en un produit (I) contenant l'ester 2-éthylhexylique de l'acide (méth)acrylique, le 2-éthylhexanol restant et l'acide (méth)acrylique restant et en un produit (II) contenant le catalyseur et des composants à point d'ébullition plus élevé que celui de l'ester 2-éthylhexylique de l'acide (méth) acrylique.

11. Procédé selon la revendication 10, caractérisé en ce que l'unité de rectification (I) est une colonne de rectification (I), que le mélange de produits extrait de la zone de réaction est introduit dans la partie inférieure de la colonne de rectification (I), que le produit (II) est extrait du fond de la colonne de rectification (I) et le produit (I) est extrait à la tête de la colonne de rectification (I), et qu'une partie du produit (II) est recyclée dans la zone de réaction dans le premier secteur de réaction directement et/ou par l'intermédiaire de l'unité de rectification (III) .

12. Procédé selon la revendication 11, caractérisé en ce qu'une partie du produit (II) est extraite et introduite dans une unité de distillation supplémentaire (IV) et dans celle-ci, elle est séparée en un produit (III) contenant le 2-éthylhexanol, l'acide (méth) acrylique et l'ester 2-éthylhexylique de l'acide (méth)acrylique et en un produit (IV) contenant le catalyseur ainsi que des composants à point d'ébullition plus élevé que celui de l'ester 2-éthylhexylique de l'acide (méth)acrylique, le produit (III) est recyclé dans l'unité de rectification (I) et/ou dans la zone de réaction, le catalyseur est séparé du produit (II) et/ou du produit (IV) par extraction avec de l'eau et la phase aqueuse produite est recyclée dans la zone de réaction et pour l'extraction l'on utilise une partie de la phase aqueuse produite dans l'unité de rectification (III).

13. Procédé selon la revendication 12, caractérisé en ce que le produit (I) retiré de l'unité de rectification (I) est introduit dans une unité de rectification (II) et séparé dans celle-ci en un produit (V) contenant le 2-éthylhexanol restant, de l'acide (méth)acrylique et des composants à point d'ébullition plus bas que celui du (méth) acrylate de 2-éthylhexyle, du (méth)acrylate de 2-éthylhexyle et en un produit (VI) à point d'ébullition plus élevé que celui du (méth)acrylate de 2-éthyle.

14. Procédé selon la revendication 13, caractérisé en ce que le produit (V) est recyclé dans la zone de réaction, le produit (VI) est recyclé dans l'unité de rectification (I), l'unité de rectification (II) est une colonne de rectification (II), et le produit (V) dans la partie supérieure de la colonne de rectification (II) et le produit (VI) du fond de la colonne de rectification (II) et le (méth)acrylate de 2-éthylhexyle sont séparés dans la partie inférieure de la colonne de rectification (II) en tant que produit secondaire soutiré en phase vapeur.
